# EUROPEAN PATENT SPECIFICATION

(11) **EP 0 367 575 B1**
(45) Date of publication and mention of the grant of the patent: **06.04.1994**
(21) Application number: 89311263.1
(22) Date of filing: 31.10.1989
(51) Int. Cl.: C12N 15/28, C12P 21/02, C07K 13/00, A61K 37/02, C12N 1/00

(54) **Human lymphotoxin**
Menschliches Lymphotoxin
Lymphotoxine humaine

(30) Priority: 31.10.1988 JP 275732/88
(43) Date of publication of application: 09.05.1990
(73) Proprietor: Sankyo Company Limited, Tokyo (JP)
(72) Inventor: Uesugi, Seiichi, Minoo-city, Osaka-prefecture (JP); Takeda, Ken, Yokohama-city, Kanagawa-prefecture (JP)
(74) Representative: Gibson, Christian John Robert

(56) References cited:
- EP-A- 0 164 965
- EP-A- 0 232 107
- EP-A- 0 250 000

## Description

The present invention relates to N-terminal deletion mutants of human lymphotoxin, preparations thereof, uses therefor, methods of preparation thereof, nucleic acid sequences therefor, and antibodies and preparations thereof.

Human lymphotoxin (HL) was first identifed as long ago as 1968 [C.F. Ruddle et al., J. Exp. Med. (1969), 128, 1267 - 1279]. It is one of the family of lymphokines, which includes the interferons and tumour necrosis factors. In common with other lymphokines, human lymphotoxin has a wide range of antitumour activities and has little or no effect on normal cell lines. However, also in common with other lymphokines, human lymphotoxin is naturally produced in only very small amounts.

It has been reported that human lymphotoxin is a glycoprotein having a relative molecular mass (Mᵣ) of between 60,000 and 70,000, as measured by molecular sieve chromatography [Nature, 312, pp.721 - 4, (1984)]. The monomeric lymphotoxin only has an Mᵣ of 25,000, and other reported values go as low as 20,000. This range of weights is owing to N-linked glycosylation. Although this glycosylation occurs naturally, it has been observed to have little effect on cytotoxic activity, as recombinant lymphotoxin, which has no glycosylation, has approximately the same activity as natural lymphotoxin.

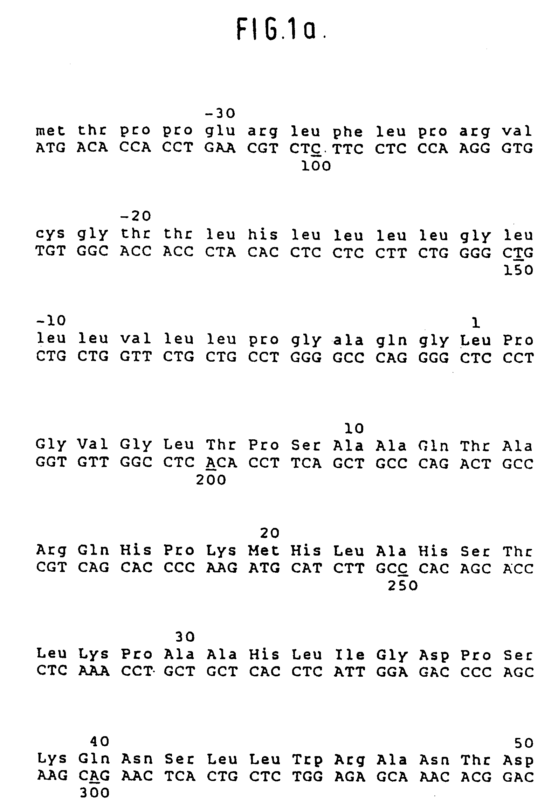

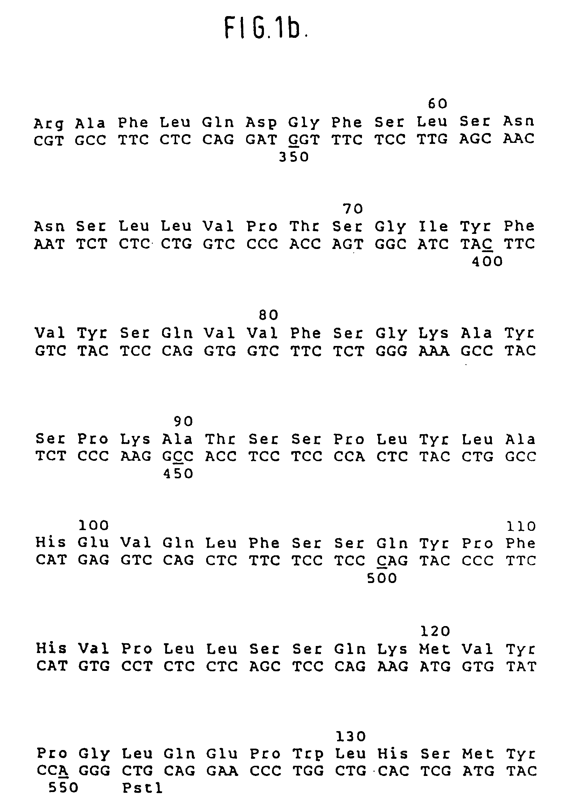

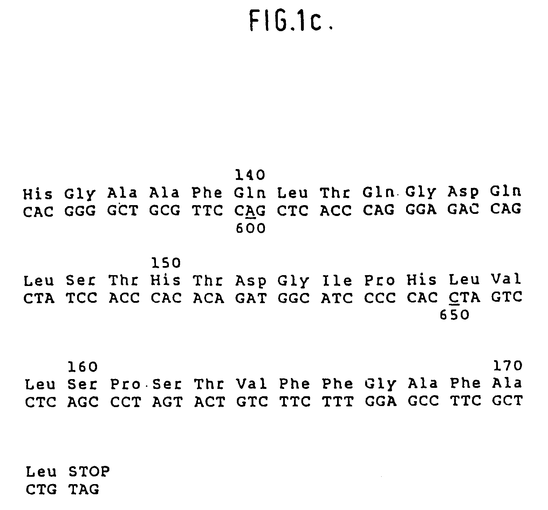

The human lymphotoxin amino acid sequence and the corresponding cDNA are shown in Fig. 1 of the accompanying drawings, and have been previously reported (Nature, supra). The cDNA sequence corresponding to natural human lymphotoxin mRNA has no restriction enzyme cleavage sites, which is inconvenient for protein engineering.

Accordingly, the nucleotide sequence of the cDNA was modified to generate the following characteristics without changing the encoded peptide sequence:
(1) recognition sites (one each) for BssHI, EcoRI, KpnI, HindIII, XhoI, SacI, SmaI, HpaI, BamHI and PstI;
(2) elimination of direct repeats in 100 nucleotide blocks by codon alteration; and
(3) elimination of palindromic sequences exceeding 6 base pairs in any 30-base sequence.

The modified cDNA has been expressed in E. coli, yielding active human lymphotoxin [Abstracts of Programs and Lectures, The 10th Annual Meeting of The Society of Molecular Biology of Japan, (1987), p.104].

Attempts to obtain N-terminal deletion mutants of lymphokines such as, for example, human tumour necrosis factor and human lymphotoxin, by genetic manipulation, have led to reports that certain N-terminal deletion mutants exhibit higher specific activities than does the complete recombinant molecule [c.f. Cancer Res., (1987), 47, 145-149; Seikagaku, (1987), 59, No.8, p.930]. This led to the finding that mutants of human lymphotoxin lacking 11, 19, 22, 24 and 26 N-terminal amino acids exhibit specific activities equal to or slightly higher than the complete molecule (c.f. Seikagaku, supra).

The N-terminal modification of human lymphotoxin has, so far, been of a random nature, and has uniformly yielded results indicating that N-terminal deletion mutants have comparable activity to the parent molecule. To achieve these mutants, 5′-digestion of the synthetic gene was effected, and no apparent indication was obtained to suggest that any particular N-terminal deletion mutant would provide any advantage over any other.

Surprisingly, therefore, it has now been discovered that the N-terminal deletion mutant lacking the initial 27 amino acids has considerably better activity than the naturally occurring molecule or, indeed, any other N-terminal deletion mutant. Also, as the mutant lacking the 28mer has no apparent activity, it may be conjectured that the mutant lacking the 27mer represents the optimal N-terminal deletion, from the view of the removal of unnecessary amino acid residues.

The present inventors have provided a human lymphotoxin molecule having the N-terminal artificially deleted by genetic manipulation, and a synthetic gene therefor. The deletion of the N-terminal 27mer yields a human lymphotoxin having significantly enhanced antitumour activity.

Thus, in a first aspect, the present invention provides lymphotoxin which is physiologically active in humans, and lacking the N-terminal 27mer of the naturally occurring molecule; precursors therefor; and physiologically active derivatives thereof. Such derivatives include, for example, salts and esters.

By '27mer' is meant that sequence of 27 amino acid residues forming the N-terminal portion of the naturally occurring human lymphotoxin molecule, as identified in Fig. 1 at residues 1-27 inclusive.

The invention is further discussed below with reference, where appropriate, to the accompanying drawings, in which:
Fig. 1 shows the nucleotide sequence of the known human lymphotoxin cDNA,
Fig. 2 shows the modified cDNA nucleotide sequence for human lymphotoxin, with no N-terminal deletion;
Fig. 3 shows a general scheme for constructing plasmids encoding N-terminal HL deletion mutants;
Fig. 4 shows a number of synthetic oligonucleotides encoding various N-terminal oligopeptides of HL;
Figs. 5a and 5b show SDS PAGE gels of whole HL and HL N-terminal deletion mutants; and
Figs. 6a and 6b show SDS PAGE gels of the respective E. coli crude extracts.

The human lymphotoxin (HL) of the invention, provided that it lacks the 27 N-terminal amino acid residues of the naturally occurring molecule, may be modified, if desired, in any way appropriate, such as by alteration or substitution. For example, the HL of the invention may simply have a free amino terminal where the naturally occurring molecule has the 27mer, or this portion may be substituted as appropriate. As has already been described, the mutant HL lacking 27 residues (Δ27N) is more active than any other N-terminal HL deletion mutant, including that lacking only 26 residues (Δ26N). Accordingly, it is not appropriate to substitute the N-terminal with leucine or substituted leucine (residue 27 of the naturally occurring HL), and peptides containing this residue, as well as nucleotide sequences encoding this residue, are specifically excluded from the present invention.

Suitable N-terminal substituents will be apparent to those skilled in the art. However, for purposes of illustration, examples include: methionine; N-formylmethionine; and leader sequences and groups such as esters which are either cleaved at an appropriate stage, such as prior to, on, or after administration, to yield the free molecule, or which do not adversely affect activity, or both.

In general, it is preferred that any N-terminal substituent be so chosen as to be cleaved, degraded or otherwise removed prior to, or on, reaching the target site in the body. Such substituents may spontaneously decompose or cleave in the bodily environment (owing to change in pH conditions/ionic concentration, etc.), or may be cleaved by the action of, for example, enzymes, such as phosphatases to cleave phosphate groups.

Substituents such as leader sequences will generally be a by-product of any expression system used to obtain the HL. Such substituents will generally be cleaved in the expression system, or possibly on administration, and tend to serve no particular function with regard to the physiological activity of HL, but rather tend advantageously to be involved in expression of the peptide. In the present invention, where a leader sequence is used, it may be appropriate to use the naturally occurring leader sequence (Nature, supra), but any suitable sequence may be used, especially where such has been specifically developed for a given expression system.

As used herein, the term 'peptide' means any molecule comprising 2 or more amino acids linked via a peptide bond. As such, the term includes oligopeptides, polypeptides and proteins.

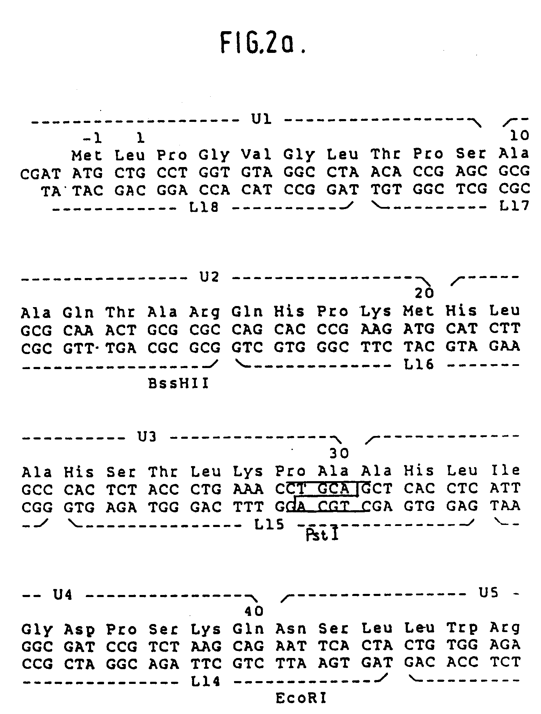

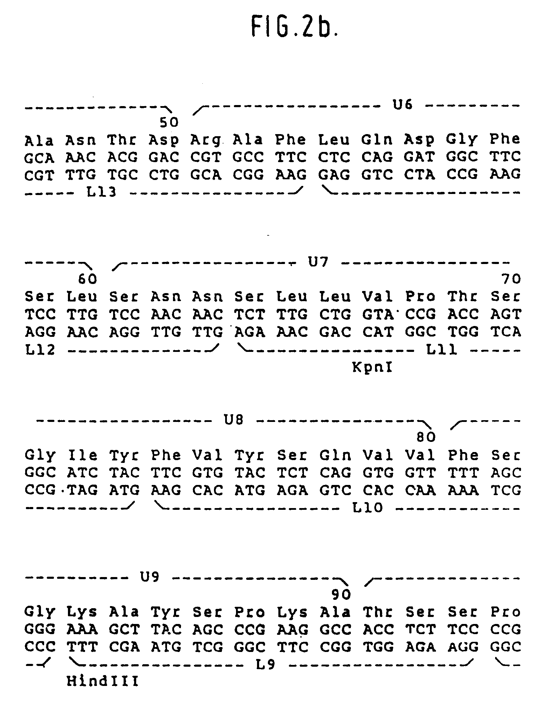

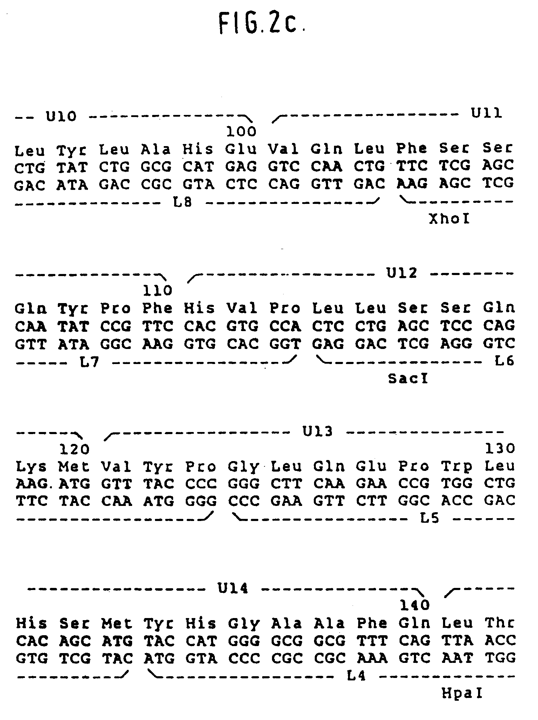

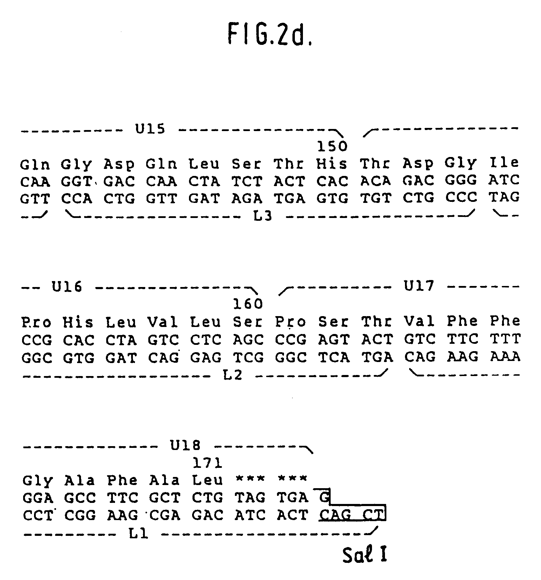

The whole HL molecule upon which the N-terminal mutants of the invention are based may be any suitable molecule, but should have the type of antitumour activity described for HL in any of the foregoing references. More specifically, the HL of the invention will preferably generally share substantial sequence homology with amino acid residues 28 et seq. of Fig. 1 or Fig. 2.

It has been found that absence of the carboxy terminal 16mer sequence yields inactive HL (Nature, supra). As such, it is preferred to provide a C-terminal sequence substantially similar to that of Fig. 1 or Fig. 2 (especially about the last 16 residues). Provided that there is no adverse effect on activity, this number is not necessarily crucial, and the residues may also be substituted by their equivalents, if desired. It is particularly preferred to retain the terminal leucine, as this residue may be important to activity.

In general, it will be appreciated that the activity of any given protein is dependent upon certain conserved regions of the molecule, while other regions have little importance associated with their particular sequence, and may be virtually or completely redundant. Accordingly, the present invention also includes any variants and mutants on the sequence which still show substantial HL activity. Such variants and mutants include, for example, deletions, additions, insertions, inversions, repeats and type-substitutions (e.g. substituting one hydrophilic residue for another, but not strongly hydrophilic for strongly hydrophobic as a rule). Small changes will generally have little effect on activity, unless they are an essential part of the molecule, and may be a side-product of genetic manipulation, for example, when generating extra restriction sites, if such is desired.

It will be appreciated that the coding sequence may be modified in any manner desired, provided that there is no adverse effect on HL activity. Spot mutations and other changes may be effected to add or delete restriction sites, for example, to otherwise assist in genetic manipulation/expression, or to enhance or otherwise conveniently to modify the HL molecule.

As used herein, the term 'adverse effect' means any effect on activity (or other properties, depending on its intended use) which renders the molecule only as effective as, or less effective than, the naturally occurring HL identified above. This term does not include modifications effectively nullified by the time the active site is reached.

The C-terminal of the HL of the invention does not form an essential feature of the present invention, except insofar as it is necessary for activity. If desired, the carboxyl group may be substituted or modified in any manner apparent to those skilled in the art. Such substitutions may include the formation of salts and esters, for example, or any other substitution as appropriate. Modification may include the deletion of one or more C-terminal amino acid residues, partially or entirely, provided that this has no adverse effect on activity. In particular, it is likely that the terminal leucine is necessary for activity, and so it is preferred to delete, add to, or change if desired, the residues in the terminal region other than leucine. Deletion of the terminal carboxyl group may be useful in preventing undesirable reactions, which purpose may also be served by the use of an appropriate protecting group, for example. Modification may also include replacement of one or more of the residues with any other suitable residue, and such replacement may either be 1 : 1 or any other suitable ratio, greater or less than unity.

Modifications but, more especially, substitutions to the C-terminal may either be temporary or permanent, as with modifications and substitutions to the HL molecule as a whole. Thus, a C-terminal esterified HL may be de-esterified in vivo, either at or before reaching the target site. Likewise, the HL may be specifically modified, particularly by deletion or substitution, so as to be inactive until the target is reached, whereon activation may be internal (by enzymatic cleavage or addition, for example) or external (such as by irradiation) to activate a sensitive group.

In general, it will be appreciated that the entire molecule may be substituted or modified within wide limits. Thus, for example, it will be apparent that the HL of the invention may be heavily glycosylated without adversely affecting activity, as this is the state of naturally occurring HL. The present invention envisages both the glycosylated and the unglycosylated HL of the invention as being useful, as well as any state inbetween.

Many substitutions, additions, and the like may be effected, and the only limitation is that activity not be adversely affected. In general, an adverse effect on activity is only likely if the 3-D (tertiary) structure of the HL is seriously affected, or if an active site is somehow affected (reducing electronegativity/hydrophilicity, blocking etc.).

If it is desired to glycosylate the HL molecule selectively, rather than randomly as would be achieved by direct chemical addition, this can be achieved best by a eukaryotic, especially mammalian, system. This may either comprise a eukaryotic expression system, or treatment of the product with a suitable enzyme system in vitro, both of which are known in the art.

Selective substitution on the molecule will not generally be facile. For example, to modify only the C-terminal carboxyl group, it would most likely be necessary to block any other groups likely to be modified by the same treatment. Universal modification of a particular type of group may be acceptable, such as esterification, but it is usually acceptable and, moreover, practical to use the unmodified expression product. However, selective modification is particularly achieved by appropriate selection of expression system and/or suitable modification of the coding sequence.

Suitable substitutions, additions and the like may be effected as desired to assist in formulation, for example, or may be a product of any expression system employed.

A preferred peptide sequence is represented by the following formula (I): 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal (I)
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
(wherein N and C designate the amino and carboxy terminals, respectively, and R represents methionine, N-formylmethionine, a leader sequence or a hydrogen atom), equivalents, mutants and variants thereof, and precursors therefor.

With reference to the above peptide sequence, the term 'equivalent' is used in the sense of the preceding description, that is to say, equivalents in the sense of sequences having substitutions at the C- or N-terminals, or anywhere else, including salts and esters, and glycosylated sequences. The term 'mutants' is used with reference to deletions, additions, insertions, inversions and replacement of amino acid residues in the sequence which do not adversely affect activity. 'Variant' is used in relation to other naturally occurring human lymphotoxins which may be discovered from time to time and which share essentially the same sequence as shown in Fig. 1, but which vary therefrom in a manner to be expected within a large population. Within this definition lie allelic variation and those peptides from other species showing a similar type of activity and having a related sequence. The term 'precursor' includes such molecules as those having leader sequences or substitutions which may or may not affect activity, but which are no longer present when the HL is active, whether the effect was negated before or at the target site.

The present invention also provides nucleotide sequences encoding all or part of the HL of the invention. Where the nucleotide sequence encodes only a part of the HL, this part should at least encode the N-terminal of the invention, but at least not the leucine residue at position 27 of the naturally occurring human lymphotoxin molecule. As will be apparent from the foregoing, there is little restriction on the sequence, whether it be DNA or RNA. A gene encoding the HL of the invention may easily be reverse-engineered by one skilled in the art from the sequences given in Figures 1 and 2, together with any necessary information provided herein.

It will be appreciated that any one given reverse-engineered sequence will not necessarily hybridise well, or at all, with any given complementary sequence reverse-engineered from the same peptide, owing to the degeneracy of the genetic code. This is a factor common in the calculations of those skilled in the art, and the degeneracy of any given sequence is frequently so broad as to make it extremely difficult to synthesise even a short complementary oligonucleotide sequence to serve as a probe for the naturally occurring oligonucleotide sequence.

The degeneracy of the code is such that, for example, there may be 4, or more, possible codons for frequently occurring amino acids. Accordingly, therefore, it can be seen that the number of possible coding sequences for any given peptide can increase exponentially with the number of residues. As such, it will be appreciated that the number of possible coding sequences for the HL of the invention may have six or more figures, with little to choose between any of that number. However, it may be desirable to balance the G + C content according to the expression system concerned, and other factors may need to be taken into account which may affect the choice of coding sequence.

A preferred coding sequence is that shown in Figure 2, as it encodes the naturally occurring peptide sequence, but lacks the direct repeats of the naturally occurring gene and provides a number of restriction sites (c.f. description supra).

As stated above, hybridisation can be an unreliable indication of sequence homology but, nevertheless, those sequences showing in excess of 50%, preferably 70% and more preferably 80% homology with the sequence of Figure 2 are preferred. In each case, the sequence should encode an HL, or at least the N-terminal thereof, of the invention.

Sequences of the invention shorter than is necessary to encode the entire HL of the invention may be used, for example, either to modify the N-terminal of another peptide, especially another lymphokine, or may be used to modify, for example, an HL-encoding plasmid. Either way, the encoded peptide has the N-terminal of the invention.

The sequences of the invention may also be engineered to provide further restriction sites as may be desired. This can be done so as not to interfere with the peptide sequence of the encoded HL, or may interfere to any extent desired or necessary, provided that the final product has the necessary activity.

The nucleotide sequences of the invention are preferably sequences of DNA. Such sequences may be used alone, for example as probes, but it is generally preferred that they form part of an expression system. Thus, it is preferred that the DNA sequence form part of a vector useful in an expression system.

The general nature of vectors for use in accordance with the present invention is not crucial to the invention. In general, suitable vectors and expression vectors and constructions therefor will be apparent to those skilled in the art.

Suitable expression vectors may be based on 'phages or plasmids, both of which are generally host-specific, although these can often be engineered for other hosts. Other suitable vectors include cosmids and retroviruses, and any other vehicles, which may or may not be specific for a given system. Again, control sequences, such as recognition, promoter, operator, inducer, terminator and other sequences essential and/or useful in the regulation of expression, will be readily apparent to those skilled in the art, and may be associated with the natural HL sequence or with the vector used, or may be derived from any other source as suitable. The vectors may be modified or engineered in any suitable manner.

A particularly preferred double-stranded nucleotide sequence is as follows: 
(-1) 28 30
Met Lys Pro Ala Ala His Leu Ile Gly
CGAT ATG AAA CCT GCA GCT CAC CTC ATT GGC
TA TAC TTT GGA CGT CGA GTG GAG TAA CCG

40
Asp Pro Ser Lys Gln Asn Ser Leu Leu Trp
GAT CCG TCT AAG CAG AAT TCA CTA CTG TGG
CTA GGC AGA TTC GTC TTA AGT GAT GAC ACC

50
Arg Ala Asn Thr Asp Arg Ala Phe Leu Gln
AGA GCA AAC ACG GAC CGT GCC TTC CTC CAG
TCT CGT TTG TGC CTG GCA CGG AAG GAG GTC

60
Asp Gly Phe Ser Leu Ser Asn Asn Ser Leu
GAT GGC TTC TCC TTG TCC AAC AAC TCT TTG
CTA CCG AAG AGG AAC AGG TTG TTG AGA AAC
 
70
Leu Val Pro Thr Ser Gly Ile Tyr Phe Val
CTG GTA CCG ACC AGT GGC ATC TAC TTC GTG
GAC CAT GGC TGG TCA CCG TAG ATG AAG CAC

80
Tyr Ser Gln Val Val Phe Ser Gly Lys Ala
TAC TCT CAG GTG GTT TTT AGC GGG AAA GCT
ATG AGA GTC CAC CAA AAA TCG CCC TTT CGA

90
Tyr Ser Pro Lys Ala Thr Ser Ser Pro Leu
TAC AGC CCG AAG GCC ACC TCT TCC CCG CTG
ATG TCG GGC TTC CGG TGG AGA AGG GGC GAC

100
Tyr Leu Ala His Glu Val Gln Leu Phe Ser
TAT CTG GCG CAT GAG GTC CAA CTG TTC TCG
ATA GAC CGC GTA CTC CAG GTT GAC AAG AGC

110
Ser Gln Tyr Pro Phe His Val Pro Leu Leu
AGC CAA TAT CCG TTC CAC GTG CCA CTC CTG
TCG GTT ATA GGC AAG GTG CAC GGT GAG GAC

120
Ser Ser Gln Lys Met Val Tyr Pro Gly Leu
AGC TCC CAG AAG ATG GTT TAC CCC GGG CTT
TCG AGG GTC TTC TAC CAA ATG GGG CCC GAA

130
Gln Glu Pro Trp Leu His Ser Met Tyr His
CAA GAA CCG TGG CTG CAC AGC ATG TAC CAT
GTT CTT GGC ACC GAC GTG TCG TAC ATG GTA
 
140
Gly Ala Ala Phe Gln Leu Thr Gln Gly Asp
GGG GCG GCG TTT CAG TTA ACC CAA GGT GAC
CCC CGC CGC AAA GTC AAT TGG GTT CCA CTG

150
Gln Leu Ser Thr His Thr Asp Gly Ile Pro
CAA CTA TCT ACT CAC ACA GAC GGG ATC CCG
GTT GAT AGA TGA GTG TGT CTG CCC TAG GGC

160
His Leu Val Leu Ser Pro Ser Thr Val Phe
CAC CTA GTC CTC AGC CCG AGT ACT GTC TTC
GTG GAT CAG GAG TCG GGC TCA TGA CAG AAG

170 171
Phe Gly Ala Phe Ala Leu *** ***
TTT GGA GCC TTC GCT CTG] TAG TGA G
AAA CCT CGG AAG CGA GAC] ATC ACT CAG CT
and mutants and variants thereof, wherein the square brackets around the DNA sequence (between residues 28 and 171) indicate the preferred sequence. The encoded amino acids are shown above the sequence.

It will be appreciated that the sequence shown represents that part of a sequence necessary to encode an entire preferred HL molecule. Terminators, promoters and other such control sequences as desired may be added so as to, for example, facilitate ligation into a suitable vector, or expression, or both.

As used above, the terms 'mutant' and 'variant' have similar meanings to those used in connection with the peptide sequence, mutatis mutandis. It will be appreciated that, while a mutant or variant of a peptide sequence will always be reflected in the coding nucleotide sequence, the reverse is not necessarily true. Accordingly, it may be possible for the nucleotide sequence to be substantially changed (for example as described above with regard to degeneracy of the genetic code), without affecting the peptide sequence in any way. Such mutants and variants are within the scope of the invention.

In general, there are a number of methods which can be used to produce the peptide and nucleotide sequences of the invention. In one embodiment, the naturally occurring peptide may be produced in a conventional manner, and digested to remove the N-terminal 27mer. Such digestion may be facilitated by alteration of the nucleotide sequence to provide such as a thermo- or cold- labile site, for example, or a recognisable residue or sequence in the encoded peptide, whereat chemical cleavage, for example, or peptidase cleavage may be effected. Suitable peptidases are well known to those skilled in the art.

A straightforward method for obtaining the HL of the invention is simply to synthesise the nucleotide sequence of Figure 2, insert it into a suitable expression vector, e.g. plasmid, transform a suitable host, culture the host, and obtain the HL of the invention by any suitable means, such as sonication and centrifugation. The 'U' and 'L' designations in Fig. 2 indicate subdivisions of oligonucleotide suitable as synthetic building blocks to make up the entire coding sequence, should such a method be desired to be used.

Alternatively, the PstI-SalI, or PstI-EcoRI, fragment of pTLym, for example, can be obtained by digestion with the relevant restriction enzymes, and a suitable oligonucleotide ligated to the 5′-end coding for the missing amino acids. For ease of manipulation, it is preferred to use the PstI-SalI fragment. The resulting cDNA can then be used as above.

Other suitable methods will be apparent to those skilled in the art.

In one preferred method, plasmid pTLym containing the human lymphotoxin synthetic gene (Abstracts of Programs and Lectures, supra), is cleaved with the restriction enzymes ClaI and SalI, and two fragments are recovered. Of these fragments, the shorter fragment (about 530 bp) containing the lymphotoxin gene is separated, preferably by electrophoresis, and cleaved further with PstI, and the PstI-SalI fragment (lacking the 5'/ N-terminal) recovered.

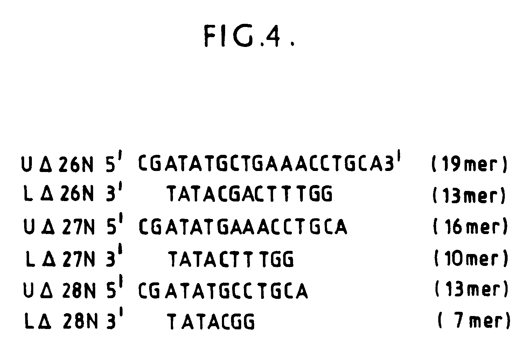

The PstI-SalI fragment encodes a peptide sequence lacking 30 amino acid residues from the N-terminal. The necessary 3 codons are then replaced using a synthetic sequence (for example, as shown in Fig. 4), having a 5' ClaI site and a 3' PstI site, annealed with a suitable complementary oligonucleotide. The annealed product is mixed with the PstI-SalI fragment, giving a ClaI-SalI fragment containing a gene coding for Δ27N (the HL of the invention). Prior to ligating this fragment back into a suitable vector, it will usually be necessary or desirable to redigest with SalI, as the exposed SalI end can self-ligate. Such redigestion may be avoided by, for example, using a different restriction site, whether by modifying the SalI site or otherwise.

Alternatively, it is possible to anneal a fragment, for example, the UΔ27N fragment (Fig. 4), to the PstI-SalI fragment, anneal this hybrid with the ClaI-SalI fragment and complete the plasmid with a ligase and a polymerase.

By phosphorylating the 5′-end of the Δ27N-encoding fragment with polynucleotide kinase and ligating with the longer ClaI-SalI fragment (about 3,730 bp), a plasmid containing the human lymphotoxin N-terminal deletion mutant synthetic gene can be prepared. Insertion of the desired gene may be confirmed by examination of the nucleotide sequence of the plasmid, for example by the method of Sanger et al. [PNAS, (1977), 74, 5463-7].

If pTLym is used as the source of the HL gene, it is possible to render the process easier for future use by deleting the PstI site in Amp^{R}. Accordingly, it would only then be necessary to digest with PstI and another suitable enzyme before ligating with the short oligonucleotide. Predigestion with PstI alone may be sufficient, but is unlikely to be efficient.

It will be appreciated that the fragment encoding the HL of the invention may easily be inserted into any suitable vector for any purpose desired. Ideally, the receiving vector has a ClaI site and a SalI site for ease of insertion, but blunt-end ligation, for example, may also be used, although this may lead to uncertainty over reading frame and direction of insertion. In such an instance, it is a matter of course to test transformants for expression, 1 in 6 of which should be useable. Suitable vectors may be selected as a matter of course by those skilled in the art according to the expression system desired.

By transforming E. coli with the plasmid obtained, selecting the transformant with ampicillin or by other suitable means, and adding tryptophan or other suitable promoter inducer (such as indoleacrylic acid), the desired human lymphotoxin N-terminal deletion mutant may be expressed. The extent of expression may be analysed by SDS polyacrylamide gel electrophoresis - SDS-PAGE (Nature, (1970), 227, pp.680 - 685).

It will also be appreciated that, where another vector is used, for example, it will be equally acceptable to employ a different selection marker or markers, or an alternative method of selection, and/or to use any suitable promoter as required or convenient.

After cultivation, the transformant cells are suitably collected, disrupted, for example sonicated, and spun-down. Disruption may also be by such techniques as enzymic digestion, using for example cellulase, or by shaking with an agent such as glass beads, but methods such as sonication are generally preferred, as no additions are necessary. The antitumour activity of the supernatant may be assayed and the amount of HL measured by SDS-PAGE, for example, allowing the specific activity to be calculated.

Conventional protein purification is suitable to obtain the expression product.

Where not specifically described herein, methods for growing and transforming cultures etc. are usefully illustrated in, for example, Maniatis (Molecular Cloning, A Laboratory Notebook, Maniatis et al. [Ed's], Cold Spring Harbor Labs, NY).

The HL of the invention is unlikely to find any use as a vaccine for humans, but may be used to generate antibodies in, for example, mice or rabbits, which antibodies can be used in assay or purification systems, for example. Such antibodies may be prepared as polyclonal or monoclonal antibodies. The method in which they are used will largely depend on the use to which they are to be put, but they may be, for example, bound to markers, such as fluorescent, enzyme or radioactive markers, left free in solution for use, for example, in Western blotting, or may be bound to a support for use, for example, in electrophoresis. Other uses for such antibodies will be apparent to those skilled in the art.

Cultures useful for the production of the HL of the invention may suitably be cultures of any living cells, and may vary from prokaryotic expression systems up to eukaryotic expression systems. One preferred prokaryotic system is that of E. coli, owing to its ease of manipulation. However, in general terms, it is preferable to express proteins intended for use in the human body in higher systems, especially mammalian cell lines. A currently preferred such system is the Chinese Hamster Ovary (CHO) cell line. Although this system tends not to be as easy to use as the E. coli system, its advantage lies in the processing of the protein after primary synthesis. E. coli, for example, does not have the equipment to glycosylate mammalian proteins, and it is preferred to glycosylate such proteins where possible, if for no other reason than that the natural proteins are glycosylated. In certain cases, glycosylation may be of no assistance whatever, and may even hinder the protein. In the present instance, glycosylation appears to serve little purpose.

Other expression systems which may be employed include streptomycetes, for example, and yeasts, such as Saccharomyces spp., especially S. cerevisiae. With current progress in research, other systems, such as B. subtilis and others, are becoming available and there is no effective limit on which system is used, provided that it is suitable. The same systems may also be used to amplify the genetic material, but it is generally convenient to use E. coli for this purpose where only proliferation of the DNA is required.

As stated above, the HL of the invention has particularly potent antitumour activity. It is conceivable that this HL could be used without any prior preparation, but it is generally preferable that it is formulated in a suitable manner prior to use. Owing to the extremely high activity of HL, only vanishingly small traces need to be employed for any one particular application, especially where the HL is delivered straight to the target site (for example in vitro applications and delivery by catheter).

Suitable formulations for administration to the patient will generally be for administration by injection, as formulations administered via the digestive tract will tend to leave the HL open to attack by digestive enzymes. This route is also not favoured because of the small amounts of HL used which could effectively vanish in the digestive tract without ever entering the blood stream.

Thus, suitable formulations will generally be for administration by routes not involving the digestive tract. The small amounts of HL necessary will tend to exclude formulations, such as transdermal patches, which require relatively large amounts of active substance, and favour formulations by such routes as eye drops and injections, for example. However, such formulations may be used, if desired.

Such direct applications as eye drops and injections do not generally require ingredients such as flavourings, but it may be advantageous to include salts and other additives to render the formulation isotonic with the body fluids, and/or additives to assist in preservation of HL during storage (for example polyethylene glycol), and other additives, such as enzyme inhibitors and bacteriostatic agents. These and other ingredients which may be of use, such as diluents and surfactants, will be apparent to those skilled in the art.

Other suitable formulations for administration of the HL of the invention include, for example, implants. Such implants may take the form of a biodegradable plastic or resin in which the HL is embedded. As the plastic or resin decomposes, the HL is released into the blood stream, or the surrounding tissues. Such implants are generally placed just below the skin, for convenience, but they may also be situated closer to the desired site, typically by surgery.

As mentioned above, the HL of the invention may be directly applied to the target site and, typically, formulations for such administration will tend to be considerably more concentrated than those intended for injection. However, similar considerations regarding shelf-life and the like still pertain.

Formulations for injection may also involve, for example, taking an antibody which is specific for an antigen known to be in some way associated with the target, and linking the antibody to the HL of the invention. Alternatively, liposomes may be used. Either of these methods is intended to assist the HL in reaching its target. A further suitable modification to the HL in such formulations is to attach a highly radioactive group thereto, the result of which is to effectively provide a double-headed arrow, the HL recognising the tumour and helping to kill the tumour, the radioactive moiety also serving to help kill the tumour by lethal radiation.

The present invention also envisages methods for the treatment of animals in need thereof, such animals preferably being mammals, and most preferably being human beings. The treatment will tend to comprise administration of non-toxic formulations described above in the appropriate manner and in suitable doses.

While a single dose may be applied, it is generally preferable to administer one or more doses over a period of time, for example, three doses a week may be sufficient. In any event, the required dosage will depend on such factors as the age, weight and general condition of the patient. Useful regimens will be readily apparent to those skilled in the art, although a daily single or divided dose of about 1 - 1,000 µg/kg for adults is preferred.

The following Examples serve to illustrate the invention, and are not to be construed as limiting in any way the scope thereof.

### EXAMPLES

### (1) Construction of Plasmids Containing Synthetic Genes Encoding HL N-Terminal Deletion Mutants

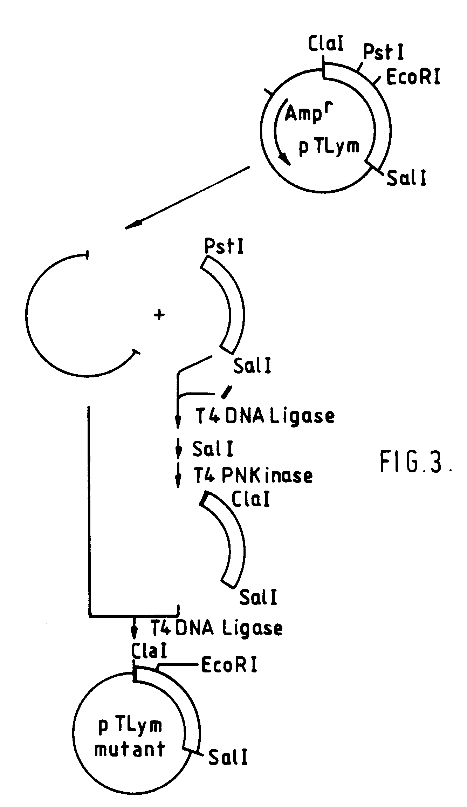

In the present invention, plasmids (hereinafter called pTLymΔ25N, pTLymΔ26N, pTLymΔ27N, pTLymΔ28N and pTLymΔ37N respectively) containing genes coding for mutants having 25, 26, 27, 28 and 37 amino acids deleted respectively from the N-terminal of human lymphotoxin (hereinafter called Δ25N, Δ26N, Δ27N, Δ28N and Δ37N respectively) were constructed. The procedures followed were as shown in Fig. 3 (wherein the blocked in '/' indicates a double-stranded oligomer). The construction of pTLymΔ26N is now described.

The two fragments obtained by cleaving pTLym with ClaI and SalI (Abstracts of Programs and Lectures, supra, Japanese Patent Application No. 287035/1987) were recovered by electrophoresis. The shorter fragment of about 530 bp containing the human lymphotoxin gene (shown in Fig. 2) was further cleaved with PstI, and the PstI-SalI fragment encoding the N-terminal deletion (Fig. 2) was recovered by electrophoresis.

75 - 100 pmol (0.01 A₂₆₀ Unit) of UΔ26N and LΔ26N (encoding amino acids 27 - 30, Fig. 4), phosphorylated at the 5′-end were annealed in 6.6 mM Tris-HCℓ buffer (pH7.6, 1mM spermidine, 100 mM MgCℓ₂, 15mM DTT and 0.2 mg/ml Bovine Serum Albumin), followed by addition of 0.5 pmol of the PstI-SalI fragment and 350 units of T4 DNA ligase. The ligation reaction was carried out at 15°C for 12 hours.

After heat treatment at 70°C for 15 minutes to inactivate enzyme (other treatments, such as the use of heavy metal ions, cyanide or phenol, may also be used, but it is then necessary to remove the inactivated enzyme), and ethanol precipitation, 25 units of SalI were added, and the cleavage reaction was carried out at 37°C for 12 hours. The product was then subjected to PAGE using a 5% gel, followed by isolation and purification to give the ClaI-SalI fragment coding for Δ26N.

To 0.3 pmol of this fragment were added 10 units of polynucleotide kinase, and the reaction was carried out at 37°C for one hour to phosphorylate the 5′-end. The reaction mixture was mixed with 0.1 pmol of the longer pTLym ClaI-SalI fragment obtained previously, followed by addition of 350 units of T4 DNA ligase (Takara, Syuzo Co. Ltd.), and ligation was carried out at 20°C for 12 hours.

E. coli HB101 was transformed and grown up (c.f. Molecular Cloning, A Laboratory Notebook, Maniatis et al. [Ed's], Cold Spring Harbor Labs, NY) and pTLymΔ26N was subsequently recovered. Insertion of the desired gene was confirmed by determination of the base sequence of the DNA by the method of Sanger et al. (supra).

pTLymΔ25N, pTLymΔ27N and pTLymΔ28N were constructed by the same technique, but using appropriate synthetic oligonucleotide couples, of which those used for pTLymΔ27N and pTLymΔ28N (UΔ27N/LΔ27N and UΔ28N/LΔ28N) are shown in Fig. 4.

Δ37N was produced by a similar technique to the above but by digestion with EcoR1 in the place of PstI followed by the addition of a 4-codon oligonucleotide as follows:

UΔ37N 5' CGATATGTCTAAGCAG (16mer)
LΔ37N 3' TATACAGATTCGTCTTAA (18mer).

### (2) Expression of N-Terminal Deletion Mutants

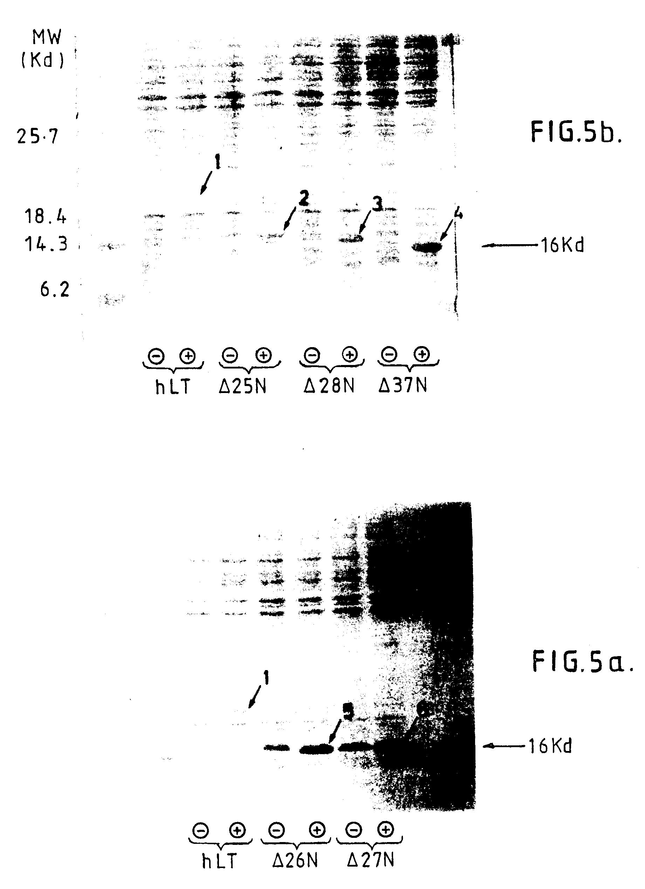

Cultures of E. coli HB101 transformed with pTLymΔ26N, pTLymΔ27N and pTLymΔ28N were grown in 5 ml of LB medium (Bacto tryptone 10 g, Bacto yeast extract 5 g and NaCℓ 10 g/l) containing 40 µg/ml of ampicillin, at 37°C for 8 to 10 hours. 50 µℓ of broth was transferred into 5 ml of M9 medium (Na₂HPO₄ 6 g, K₂HPO₄ 3 g, NaCℓ 0.5 g, NH₄Cℓ 1 g and 10 ml 0.01M CaCℓ₂/l) containing 0.2% (w/v) Casamino acids and 40 µg/ml ampicillin, and cultivated at 37°C for 10 to 16 hours. 2 ml of this broth were added to 200 ml of M9 medium, and cultivation was carried out at 37°C. After 1.5 hours, indoleacrylic acid (IAA) was added, to a final concentration of 40 µg/ml, to induce the tryptophan promoter. After 24 hours, the cells were collected, thoroughly boiled with SDS-containing Sample Buffer (0.0625M Tris-HCℓ (pH 6.8), 2% SDS, 10% glycerol, 5% mercaptoethanol and 0.001% bromophenol blue) and run on a 15% SDS polyacrylamide gel. The results for Δ26N and Δ27N are shown in Fig. 5a, and for Δ25N, Δ28N and Δ37N in Fig. 5b. Calculating the molecular weights of the N-terminal deletion mutants, the bands induced with IAA show the recombinant with no deletion, and the N-terminal deleted body (arrowheads: hLT/1 - recombinant with no deletion, 2 - Δ25N, 3 - Δ28N, 4 - Δ37N, 5 - Δ26N, 6-Δ27N, 16K - molecular weight).

### (3) Preparation of Crude Extract

E. coli cells collected after cultivation were washed with 20 ml of 20 mM Tris-HCℓ buffer (pH 8.0) containing 30 mM NaCℓ and suspended in a crushing buffer (20 mM Tris-HCℓ (pH 8.0), 30 mM NaCℓ, 1 mM EDTA, 5 mM β-mercaptoethanol). Sonication was effected 12 times for 1 minute at intervals of 2 minutes (λ 70 µm) to disrupt the cells. The supernatant was recovered from the crude extract after centrifugation at 15,000 rpm for one hour.

### (4) Quantitation

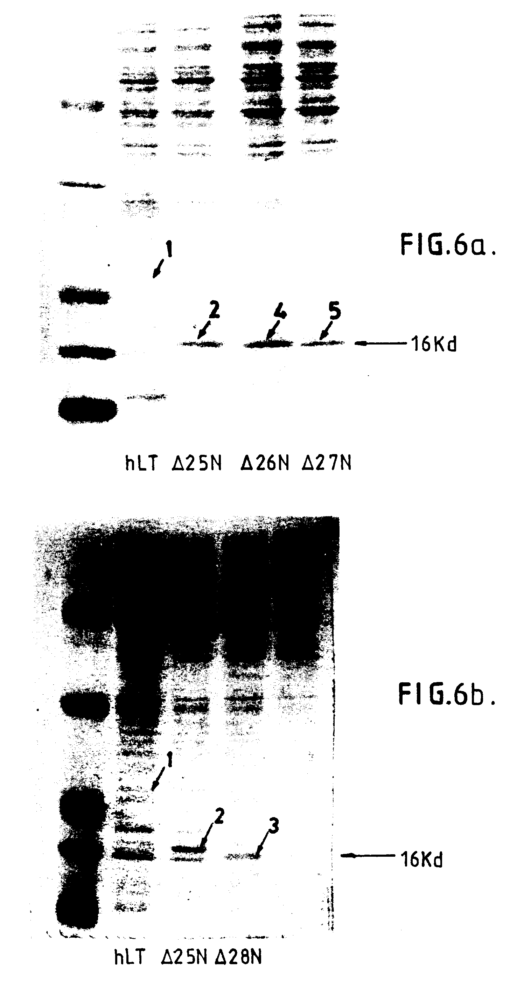

The crude extract was electrophoresed by SDS PAGE using a 15% gel. The results are shown in Fig. 6a and Fig. 6b (arrowheads: hLT/1 - recombinant with no deletion, 2 - Δ25N, 3 - Δ28N, 4 - Δ26N, 5 - Δ27N and 16K - molecular weight). As Δ37N showed no measurable HL activity, the crude extract was not prepared.

The concentrations of the bands were measured by dual-wavelength TLC scanner CS-900 (manufactured by Shimazu Seisakusho) and the relative ratios of the respective human lymphotoxin N-terminal deletion mutants in the crude extract calculated. The values were ∼2.1% for the recombinant with no deletion, ∼5.8% for Δ25N, ∼6.8% for Δ26N, ∼6.5% for Δ27N and ∼7.6% for Δ28N.

By using rabbit antisera prepared with whole HL, recombinant protein was detected in the crude extract of Example (2), while no recombinants were observed in the control E. coli containing only pBR322.

### (5) Partial Purification of Δ27N

E. coli HB101 transformed with pTLymΔ27N was cultured in 5 litres of M9 medium [containing 0.2% (w/v) Casamino acids and 40 µg/ml ampicillin] for 24 hours. The cells were collected and suspended in crushing buffer as described in (3) above. The suspension was sonicated and a supernatant obtained, to which was added streptomycin at 4°C for 30 minutes to a final concentration of 1%. The mixture was left to stand for 30 minutes, and then centrifuged at 7,000 rpm for 20 minutes to remove precipitates, including nucleic acids.

Ammonium sulphate to a final concentration of 60% was added to the supernantant with ice-cooling for 30 minutes. This was left to stand, with ice-cooling for 30 minutes, and then centrifuged at 7,000 rpm for 20 minutes to recover the precipitate.

The pellet was dissolved in 30 ml of Q-buffer (20 mM Tris-HCℓ (pH 8.5)), and then thoroughly dialysed against Q-buffer. After dialysis, a sample was loaded on a Q-Sepharose Fast Flow (manufactured by Pharmacia) column (φ2.4 x 13.5 cm). A sodium chloride gradient of 0 - 2M (prepared with 300 ml x 300 ml of Q-buffer containing 0M and 2M NaCℓ respectively) was eluted with Q-buffer, with a flow rate of 1 ml/min.

Measurement of the lymphotoxin activities of the respective fractions confirmed that Δ27N was in the fraction eluted at a sodium chloride concentration of about 0.1 M.

To this fraction was added ammonium sulphate with ice-cooling for 30 minutes to a final concentration of 60%. After being left to stand with ice-cooling for 30 minutes, the mixture was subjected to centrifugation at 15,000 rpm for 20 minutes to obtain a precipitate. The precipitate was dissolved in 10 ml of G-buffer [20 mM Tris-HCℓ (pH 8.0), 0.1 M NaCℓ], and further dialysed thoroughly against G-buffer.

After dialysis, a sample was run on a Sephadex G-75 (Pharmacia) column (φ2.2 x 87 cm). SDS PAGE as described in (2) above confirmed that Δ27N was eluted in the fraction of 145 to 172 ml (Kd = 0.261).

14 mg of Δ27N (90% purity) was obtained.

### TEST EXAMPLES

Determination of antitumour activity was by the method of B.B. Aggarwal (J. Bol. Chem., 260, No. 4, pp. 2,345-54).

Each human lymphotoxin crude extract described in (3) was diluted stepwise from 0.1 mg to 10⁻⁶ mg, and aliquots placed onto a 96-well microplate. To this were added 0.1 ml of L-929 cells (3 x 10⁵ cells/ml) and 1 µg/ml of actinomycin D, and cultivation was carried out in the presence of 5% CO₂ for 18 hours. After discarding the supernatant, the 96-well microplate was washed twice with physiological saline (0.9% (w/v) NaCℓ), and 100 µℓ of Crystal Violet (methanol : water 1 : 4 by volume) was apportioned to each well and stood for 20 minutes.

After discarding the Crystal Violet solution, the microplate was washed 3 times with physiological saline, 200 µℓ of a 1% (w/v) SDS solution was added, and the plate was allowed to stand for 30 minutes.

The absorbance of the solution was measured at 590 nm, to determine activity. Lymphotoxin activity corresponding to 50% of the absorbance of the Control is defined as one unit.

The total protein of the crude extracts was measured by conventional means.

The activity per total protein content was calculated, and from the results in (4) above specific activities of the respective human lymphotoxins could be determined. The results are shown in Table 1.

Δ27N was found to have a specific activity of about 13.5-fold higher than that of the recombinant with no deletion.

**TABLE 1**

| | Activity of total protein in crude extract (unit/mg protein) | Specific activity (unit/mg protein) |
|---|---|---|
| Complete body* | 6.0 x 10⁵ | 2.8 x 10⁷ |
| Δ25N | 2.5 x 10⁶ | 4.3 x10⁷ |
| Δ26N | 1.9 x 10⁷ | 2.8 x 10⁸ |
| Δ27N | 2.5 x 10⁷ | 3.8 x 10⁸ |
| Δ28N | Not detected | - |
| Δ37N | Not detected | - |

| | | |
|---|---|---|
| * Complete body represents recombinant with no deletion. | | |

## Claims (Claims for the following Contracting State(s): AT, BE, CH, LI, DE, FR, GB, IT, LU, NL, SE)

1. Lymphotoxin which is physiologically active in humans, and lacking the N-terminal 27mer of the naturally occurring molecule.

2. A peptide according to claim 1 and having the following sequence: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 wherein:
N and C designate the amino and carboxy terminals, respectively, and R represents methionine, N-formylmethionine or a hydrogen atom,

3. A peptide according to claim 2 wherein R is hydrogen.

4. A nucleotide sequence encoding a peptide according to any preceding claim.

5. A nucleotide sequence reverse-engineered from the peptide sequence of any preceding claim and encoding that peptide.

6. A nucleotide sequence encoding a peptide as defined in claim 1 having the following sequence:

5'          AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG          3'

7. A sequence according to any of claims 4 to 6 wherein the sequence is a DNA sequence.

8. The complementary sequence of a nucleotide sequence according to any of claims 4 to 7.

9. A nucleotide sequence according to any of claims 4 to 7 in functional association with at least one control sequence therefor.

10. A nucleotide sequence according to claim 9 wherein the at least one control sequence comprises at least one operator, promoter, terminator, inducer or any suitable combination thereof.

11. A vector comprising a nucleotide sequence according to any of claims 4 to 10.

12. A vector according to claim 10 wherein the parent vector is pTLym or a mutant or variant thereof.

13. A host cell transformed with a nucleotide sequence or a vector according to any of claims 4 to 12, the cell being competent for the replication of the sequence or vector.

14. A host cell according to claim 13, the cell being a eukaryotic cell.

15. A host cell according to claim 13, the cell being a prokaryotic cell.

16. A host cell according to any of claims 13 to 15, the cell being E. coli, saccharomycetes, streptomycetes, B. subtilis or Chinese hamster ovary.

17. A method for producing a peptide having human lymphotoxin activity comprising the steps of expressing a sequence according to any of claims 4-10, or a vector according to claim 11 or 12 in a suitable expression system therefor, and purifying the desired peptide from the culture so prepared.

18. A pharmaceutical formulation comprising a peptide according to any of claims 1 to 3 in association with a physiologically acceptable carrier therefor.

19. A formulation according to claim 18 adapted for administration by injection.

20. A formulation according to claim 18 suitable for topical administration.

21. A formulation according to claim 18 suitable for administration as eye drops.

22. A formulation according to claim 18 in the form of a surgical implant.

23. A formulation according to any of claims 18 to 22 adapted for administration in divided doses.

24. A formulation according to any of claims 18 to 23 adapted to provide a dose in vivo of between about 1µg/kg and about 1000µg/kg daily.

## Claims (Claims for the following Contracting State(s): ES)

1. A method for producing a peptide comprising a lymphotoxin which is physiologically active in humans, and lacking the N-terminal 27mer of the naturally occurring molecule;
the method comprising the steps of expressing a sequence or vector encoding the peptide in a suitable expression system therefor, thereby to express the peptide, and
purifying the desired expressed peptide.

2. A method according to claim 1 wherein the prepared peptide has the following sequence: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 wherein:
N and C designate the amino and carboxy terminals, respectively, and R represents methionine, N-formylmethionine or a hydrogen atom.

3. A method according to claim 2 wherein R of the prepared peptide is hydrogen.

4. A nucleotide sequence encoding a peptide according to any preceding claim.

5. A nucleotide sequence reverse-engineered from the peptide of any of claims 1 to 3 and encoding that peptide.

6. A nucleotide sequence encoding a peptide as defined in cliam 1, having the following sequence:

5'          AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG          3'

7. A sequence according to any of claims 4 to 6 wherein the sequence is a DNA sequence.

8. The complementary sequence of a nucleotide sequence according to any of claims 4 to 7.

9. A nucleotide sequence according to any of claims 4 to 7 in functional association with at least one control sequence therefor.

10. A nucleotide sequence according to claim 9 wherein the at least one control sequence comprises at least one operator, promoter, terminator, inducer or any suitable combination thereof.

11. A vector comprising a nucleotide sequence according to any of claims 4 to 10.

12. A vector according to claim 11 wherein the parent vector is pTLym or a mutant or variant thereof.

13. A host cell transformed with a nucleotide sequence or a vector according to any of claims 4 to 12, the cell being competent for the replication of the sequence or vector.

14. A host cell according to claim 13, the cell being a eukaryotic cell.

15. A host cell according to claim 13, the cell being a prokaryotic cell.

16. A host cell according to any of claims 13 to 15, the cell being E. coli, saccharomycetes, streptomycetes, B. subtilis or Chinese hamster ovary.

17. A method for producing a peptide having human lymphotoxin activity comprising the steps of preparing a culture of cells according to any of claims 13 to 16 and purifying the desired peptide from the culture so prepared.

18. A process for the preparation of a pharmaceutical formulation comprising bringing a peptide according to any of claims 1 to 3 into association with a physiologically acceptable carrier therefor.

19. Use of a peptide defined in any of claims 1 to 3 in the preparation of an antitumour medicament.

20. A process or use according to claim 19 wherein the formulation or medicament is adapted for administration by injection.

21. A process or use according to claim 19 wherein the formulation or medicament is suitable for topical administration.

22. A process or use according to claim 19 wherein the formulation or medicament is suitable for administration as eye drops.

23. A process or use according to claim 19 wherein the formulation or medicament is in the form of a surgical implant.

24. A process or use according to any of claims 19 to 23 wherein the formulation or medicament is adapted for administration in divided doses.

25. A process or use according to any of claims 19 to 24 wherein the formulation or medicament is adapted to provide a dose in vivo of between about 1µg/kg and about 1000µg/kg daily.

## Claims (Claims for the following Contracting State(s): GR)

1. A method for producing a peptide comprising a lymphotoxin which is physiologically active in humans, and lacking the N-terminal 27mer of the naturally occurring molecule;
the method comprising the steps of expressing a sequence or vector encoding the peptide in a suitable expression system therefor, thereby to express the peptide, and
purifying the desired expressed peptide.

2. A method according to claim 1 wherein the prepared peptide has the following sequence: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 wherein:
N and C designate the amino and carboxy terminals, respectively, and R represents methionine, N-formylmethionine or a hydrogen atom.

3. A method according to claim 2 wherein R of the prepared peptide is hydrogen.

4. A method according to any preceding claim wherein the coding nucleotide sequence is reverse-engineered from the peptide of any preceding claim and encodes that peptide.

5. A method according to any preceding claim wherein the coding nucleotide sequence has the following sequence:

5'          AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG          3'

6. A method according to claim 4 or 5 wherein the sequence is a DNA sequence.

7. A method according to any of claims 1 to 6 wherein the coding nucleotide sequence is in functional association with at least one control sequence therefor.

8. A method according to claim 7 wherein the at least one control sequence comprises at least one operator, promoter, terminator, inducer or any suitable combination thereof.

9. A method according to any preceding claim wherein the cells are transformed with a vector comprising a nucleotide sequence according to any of claims 4 to 8.

10. A method according to claim 9 wherein the parent vector is pTLym or a mutant or variant thereof.

11. A host cell transformed with a nucleotide sequence or a vector defined in any of claims 4 to 10, the cell being competent for the replication of the sequence or vector.

12. A host cell according to claim 11, the cell being a eukaryotic cell.

13. A host cell according to claim 11, the cell being a prokaryotic cell.

14. A host cell according to any of claims 11 to 13, the cell being E. coli, saccharomycetes, streptomycetes, B. subtilis or Chinese hamster ovary.

15. A method for producing a peptide having human lymphotoxin activity comprising the steps of preparing a culture of cells according to any of claims 11 to 14 and purifying the desired peptide from the culture so prepared.

16. A process for the preparation of a pharmaceutical formulation comprising bringing a peptide defined in any of claims 1 to 3 into association with a physiologically acceptable carrier therefor.

17. Use of a peptide defined in any of claims 1 to 3 in the preparation of an antitumour medicament.

18. A process or use according to claim 17 wherein the formulation or medicament is adapted for administration by injection.

19. A process or use according to claim 17 wherein the formulation or medicament is suitable for topical administration.

20. A process or use according to claim 17 wherein the formulation or medicament is suitable for administration as eye drops.

21. A process or use according to claim 17 wherein the formulation or medicament is in the form of a surgical implant.

22. A process or use according to any of claims 17 to 21 wherein the formulation or medicament is adapted for administration in divided doses.

23. A process or use according to any of claims 17 to 22 wherein the formulation or medicament is adapted to provide a dose in vivo of between about 1µg/kg and about 1000µg/kg daily.

## Patentansprüche (Patentansprüche für folgende(n) Vertragsstaat(en): AT, BE, CH, DE, FR, GB, IT, LI, LU, NL, SE)

1. Im Menschen physiologisch aktives Lymphotoxin, das frei von dem N-terminalen 27mer des natürlich vorkommenden Moleküls ist.

2. Peptid gemäß Anspruch 1, das die folgende Sequenz aufweist: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 worin:
N und C jeweils den Amino- bzw. den Carboxy-Terminus bezeichnen und R für Methionin, N-Formylmethionin oder ein Wasserstoffatom steht.

3. Peptid gemäß Anspruch 2, worin R Wasserstoff ist.

4. Nukleotidsequenz, die für ein Peptid gemäß einem der vorhergehenden Ansprüche kodiert.

5. Nukleotidsequenz, die durch reverse Technik aus der Peptidsequenz eines der vorhergehenden Ansprüche erhalten wird und für dieses Peptid kodiert.

6. Nukleotidsequenz, die für ein in Anspruch 1 definiertes Peptid kodiert und die folgende Sequenz aufweist:

5'          AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG          3'

7. Sequenz gemäß einem der Ansprüche 4 bis 6, wobei die Sequenz eine DNA-Sequenz ist.

8. Zu einer Nukleotidsequenz gemäß einem der Ansprüche 4 bis 7 komplementäre Sequenz.

9. Nukleotidsequenz gemäß einem der Ansprüche 4 bis 7 in funktioneller Verbindung mit mindestens einer Kontrollsequenz dafür.

10. Nukleotidsequenz gemäß Anspruch 9, wobei die mindestens eine Kontrollsequenz mindestens einen Operator, Promotor, Terminator, Induktor oder eine geeignete Kombination dieser enthält.

11. Vektor, der eine Nukleotidsequenz gemäß einem der Ansprüche 4 bis 10 enthält.

12. Vektor gemäß Anspruch 11, wobei der Eltern-Vektor pTLym oder eine Mutante oder eine Variante davon ist.

13. Mit einer Nukleotidsequenz oder einem Vektor gemäß einem der Ansprüche 4 bis 12 transformierte Wirtszelle, wobei die Zelle kompetent für die Replikation der Sequenz oder des Vektors ist.

14. Wirtszelle gemäß Anspruch 13, wobei die Zelle eine eukaryotische Zelle ist.

15. Wirtszelle gemäß Anspruch 13, wobei die Zelle eine prokaryotische Zelle ist.

16. Wirtszelle gemäß einem der Ansprüche 13 bis 15, wobei die Zelle E.coli, Saccharomycetes, Streptomycetes, B.subtilis oder chinesische Hamster-Ovarialzelle ist.

17. Verfahren zur Herstellung eines Peptids mit Human-Lymphotoxin-Aktivität, welche folgende Schritte umfaßt: Expression einer Sequenz gemäß einem der Ansprüche 4 bis 10 oder eines Vektors gemäß Anspruch 11 oder 12 in einem dafür geeigneten Expressionssystem und Reinigen des gewünschten Peptids aus der so hergestellten Kultur.

18. Arneimittelzubereitung, enthaltend ein Peptid gemäß einem der Ansprüche 1 bis 3 in Verbindung mit einem physiologisch geeigneten Trägermaterial dafür.

19. Zubereitung gemäß Anspruch 18, die für die Verabreichung durch Injektion zubereitet ist.

20. Zubereitung gemäß Anspruch 18, die für die topische Verabreichung geeignet ist.

21. Zubereitung gemäß Anspruch 18, die für die Verabreichung in Form von Augentropfen geeignet ist.

22. Zubereitung gemäß Anspruch 18 in Form eines chirurgischen Implantats.

23. Zubereitung gemäß einem der Ansprüche 18 bis 22, die für die Verabreichung in unterteilten Dosen zubereitet ist.

24. Zubereitung gemäß einem der Ansprüche 18 bis 23, die so zubereitet ist, daß sie in vivo eine Dosis zwischen etwa 1µg/kg und etwa 1.000µg/kg pro Tag bereitstellt.

## Patentansprüche (Patentansprüche für folgende(n) Vertragsstaat(en): ES)

1. Verfahren zur Herstellung eines Peptids, welches ein Lymphotoxin darstellt, das im Menschen physiologisch aktiv ist und das frei von dem N-terminalen 27mer des natürlich vorkommenden Moleküls ist, wobei das Verfahren folgende Schritte umfaßt : Expression einer Sequenz oder eines Vektors, die bzw. der für das Peptid kodiert, in einem dafür geeigneten Expressionssystem, wobei das Peptid exprimiert wird, und Reinigen des gewünschten exprimierten Peptids.

2. Verfahren gemäß Anspruch 1, wobei das hergestellte Peptid die folgende Sequenz aufweist: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 worin:
N und C jeweils den Amino- bzw. den Carboxy-Terminus bezeichnen und R für Methionin, N-Formylmethionin oder ein Wasserstoffatom steht.

3. Verfahren gemäß Anspruch 2, wobei R in dem hergestellten Peptid Wasserstoff ist.

4. Nukleotidsequenz, die für ein Peptid gemäß einem der vorhergehenden Ansprüche kodiert.

5. Nukleotidsequenz, die durch reverse Technik aus der Peptidsequenz eines der Ansprüche 1 bis 3 erhalten wird und für dieses Peptid kodiert.

6. Nukleotidsequenz, die für ein in Anspruch 1 definiertes Peptid kodiert und die folgende Sequenz aufweist:

5'            AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG                3'

7. Sequenz gemäß einem der Ansprüche 4 bis 6, wobei die Sequenz eine DNA-Sequenz ist.

8. Zu einer Nukleotidsequenz gemäß einem der Ansprüche 4 bis 7 komplementäre Sequenz.

9. Nukleotidsequenz gemäß einem der Ansprüche 4 bis 7 in funktioneller Verbindung mit mindestens einer Kontrollsequenz dafür.

10. Nukleotidsequenz gemäß Anspruch 9, wobei die mindestens eine Kontrollsequenz mindestens einen Operator, Promotor, Terminator, Induktor oder eine geeignete Kombination dieser enthält.

11. Vektor, der eine Nukleotidsequenz gemäß einem der Ansprüche 4 bis 10 enthält.

12. Vektor gemäß Anspruch 11, wobei der Eltern-Vektor pTLym oder eine Mutante oder eine Variante davon ist.

13. Mit einer Nukleotidsequenz oder einem Vektor gemäß einem der Ansprüche 4 bis 12 transformierte Wirtszelle, wobei die Zelle kompetent für die Replikation der Sequenz oder des Vektors ist.

14. Wirtszelle gemäß Anspruch 13, wobei die Zelle eine eukaryotische Zelle ist.

15. Wirtszelle gemäß Anspruch 13, wobei die Zelle eine prokaryotische Zelle ist.

16. Wirtszelle gemäß einem der Ansprüche 13 bis 15, wobei die Zelle E.coli, Saccharomycetes, Streptomycetes, B.subtilis oder chinesische Hamster-Ovarialzelle ist.

17. Verfahren zur Herstellung eines Peptids mit Human-Lymphotoxin-Aktivität, welche folgende Schritte umfaßt: Herstellen einer Kultur von Zellen gemäß einem der Ansprüche 13 bis 16 und Reinigen des gewünschten Peptids aus der so hergestellten Kultur.

18. Verfahren zur Herstellung einer Arzneimittelzubereitung, das darin besteht, daß ein Peptid gemäß einem der Ansprüche 1 bis 3 mit einem physiologisch geeigneten Trägermaterial dafür kombiniert wird.

19. Verwendung eines in einem der Ansprüche 1 bis 3 definierten Peptids zur Herstellung eines Antitumor-Arzneimittels.

20. Verfahren oder Verwendung nach Anspruch 19, wobei die Zubereitung oder das Arzneimittel für die Verabreichung durch Injektion zubereitet ist.

21. Verfahren oder Verwendung nach Anspruch 19, wobei die Zubereitung oder das Arzneimittel für die topische Verabreichung geeignet ist.

22. Verfahren oder Verwendung nach Anspruch 19, wobei die Zubereitung oder das Arzneimittel zur Verabreichung in Form von Augentropfen geeignet ist.

23. Verfahren oder Verwendung nach Anspruch 19, wobei die Zubereitung oder das Arzneimittel in Form eines chirurgischen Implantats ist.

24. Verfahren oder Verwendung nach einem der Ansprüche 19 bis 23, wobei die Zubereitung oder das Arzneimittel zur Verabreichung in unterteilten Dosen zubereitet ist.

25. Verfahren oder Verwendung nach einem der Ansprüche 19 bis 24, wobei die Zubereitung oder das Arzneimittel so zubereitet wird, daß in vivo eine Dosis zwischen etwa 1 µg/kg und etwa 1000 µg/kg bereitgestellt wird.

## Patentansprüche (Patentansprüche für folgende(n) Vertragsstaat(en): GR)

1. Verfahren zur Herstellung eines Peptids, welches ein Lymphotoxin darstellt, das im Menschen physiologisch aktiv ist und das frei von dem N-terminalen 27mer des natürlich vorkommenden Moleküls ist, wobei das Verfahren folgende Schritte umfaßt : Expression einer Sequenz oder eines Vektors, die bzw. der für das Peptid kodiert, in einem dafür geeigneten Expressionssystem, wobei das Peptid exprimiert wird, und Reinigen des gewünschten exprimierten Peptids.

2. Verfahren gemäß Anspruch 1, wobei das hergestellte Peptid die folgende Sequenz aufweist: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 worin:
N und C jeweils den Amino- bzw. den Carboxy-Terminus bezeichnen und R für Methionin, N-Formylmethionin oder ein Wasserstoffatom steht.

3. Verfahren gemäß Anspruch 2, wobei R in dem hergestellten Peptid Wasserstoff ist.

4. Verfahren gemäß einem der vorhergehenden Ansprüche, wobei die kodierende Nukleotidsequenz durch reverse Technik aus dem Peptid eines der vorhergehenden Ansprüche erhalten wird und für dieses Peptid kodiert.

5. Verfahren nach einem der vorhergehenden Ansprüche, wobei die kodierende Nukleotidsequenz die folgende Sequenz aufweist:

5' AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG 3'

6. Verfahren nach Anspruch 4 oder 5, wobei die Sequenz eine DNA-Sequenz ist.

7. Verfahren nach einem der Ansprüche 1 bis 6, wobei die kodierende Nukleotidsequenz in funktioneller Verbindung mit mindestens einer Kontrollsequenz dafür ist.

8. Verfahren nach Anspruch 7, wobei die mindestens eine Kontrollsequenz mindestens einen Operator, Promotor, Terminator, Induktor oder eine geeignete Kombination dieser enthält.

9. Verfahren nach einem der vorhergehenden Ansprüche, wobei die Zellen mit einem Vektor transformiert werden, der eine Nukleotidsequenz gemäß einem der Ansprüche 4 bis 8 enthält.

10. Verfahren gemäß Anspruch 9, wobei der Eltern-Vektor pTLym oder eine Mutante oder eine Variante davon ist.

11. Mit einer Nukleotidsequenz oder einem Vektor gemäß einem der Ansprüche 4 bis 10 transformierte Wirtszelle, wobei die Zelle kompetent für die Replikation der Sequenz oder des Vektors ist.

12. Wirtszelle gemäß Anspruch 11, wobei die Zelle eine eukaryotische Zelle ist.

13. Wirtszelle gemäß Anspruch 11, wobei die Zelle eine prokaryotische Zelle ist.

14. Wirtszelle gemäß einem der Ansprüche 11 bis 13, wobei die Zelle E.coli, Saccharomycetes, Streptomycetes, B.subtilis oder chinesische Hamster-Ovarialzelle ist.

15. Verfahren zur Herstellung eines Peptids mit Human-Lymphotoxin-Aktivität, welche folgende Schritte umfaßt: Herstellen einer Kultur von Zellen gemäß einem der Ansprüche 11 bis 14 und Reinigen des gewünschten Peptids aus der so hergestellten Kultur.

16. Verfahren zur Herstellung einer Arzneimittelzubereitung, das darin besteht, daß ein Peptid gemäß einem der Ansprüche 1 bis 3 mit einem physiologisch geeigneten Trägermaterial dafür kombiniert wird.

17. Verwendung eines in einem der Ansprüche 1 bis 3 definierten Peptids zur Herstellung eines Antitumor-Arzneimittels.

18. Verfahren oder Verwendung nach Anspruch 17, wobei die Zubereitung oder das Arzneimittel für die Verabreichung durch Injektion zubereitet ist.

19. Verfahren oder Verwendung nach Anspruch 17, wobei die Zubereitung oder das Arzneimittel für die topische Verabreichung geeignet ist.

20. Verfahren oder Verwendung nach Anspruch 17, wobei die Zubereitung oder das Arzneimittel zur Verabreichung in Form von Augentropfen geeignet ist.

21. Verfahren oder Verwendung nach Anspruch 17, wobei die Zubereitung oder das Arzneimittel in Form eines chirurgischen Implantats ist.

22. Verfahren oder Verwendung nach einem der Ansprüche 17 bis 21, wobei die Zubereitung oder das Arzneimittel zur Verabreichung in unterteilten Dosen zubereitet ist.

23. Verfahren oder Verwendung nach einem der Ansprüche 17 bis 22, wobei die Zubereitung oder das Arzneimittel so zubereitet wird, daß in vivo eine Dosis zwischen etwa 1 µg/kg und etwa 1000 µg/kg bereitgestellt wird.

## Revendications (Revendications pour l'(les) Etat(s) contractant(s) suivant(s): AT, BE, CH, DE, FR, GB, IT, LI, LU, NL, SE)

1. Lymphotoxine physiologiquement active chez l'homme, qui ne possède pas le 27mère de l'extrémité N de la molécule naturelle.

2. Peptide selon la revendication 1 dont la séquence est la suivante: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 dans laquelle N et C représentent les extrémités amino et carboxy respectivement, et R représente un résidu méthionine ou N-formylméthionine ou un atome d'hydrogène.

3. Peptide selon la revendication 2 dans lequel R représente un atome d'hydrogène.

4. Séquence nucléotidique codant pour un peptide selon l'une des revendications précédentes.

5. Séquence nucléotidique construite en retour à partir de la séquence peptidique de toute revendication précédente et codant pour ce peptide.

6. Séquence nucléotidique codant pour un peptide comme défini à la revendication 1 possédant la séquence suivante:

5'          AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG          3'

7. Séquence selon l'une des revendications 4 à 6 dans laquelle la séquence est une séquence d'ADN.

8. Séquence complémentaire d'une séquence nucléotidique selon l'une des revendications 4 à 7.

9. Séquence nucléotidique selon l'une des revendications 4 à 7 associée de manière opérationnelle avec au moins une séquence de régulation.

10. Séquence nucléotidique selon la revendications 9 dans laquelle au moins une séquence de régulation comprend au moins un opérateur, promoteur, signal de terminaison, inducteur ou toute combinaison appropriée de ces séquences.

11. Vecteur comprenant une séquence nucléotidique selon l'une des revendications 4 à 10.

12. Vecteur selon la revendication 11 dans lequel le vecteur parental est pTLym ou un de ses mutants ou variants.

13. Cellule hôte transformée avec une séquence nucléotidique ou un vecteur selon l'une des revendications 4 à 12, la cellule étant compétente pour la réplication de la séquence ou du vecteur.

14. Cellule hôte selon la revendication 13, la cellule étant une cellule eucaryote.

15. Cellule hôte selon la revendication 13, la cellule étant une cellule procaryote.

16. Cellule hôte selon l'une des revendications 13 à 15, la cellule étant E. coli, saccharomyces, streptomyces, B. subtilis, ou une lignée de cellules d'ovaire de hamster chinois.

17. Procédé pour la production d'un peptide possédant l'activité de la lymphotoxine humaine comprenant les étapes consistant à exprimer une séquence selon l'une des revendications 4 à 10 ou un vecteur selon la revendication 11 ou 12 dans un système d'expression approprié et à purifier le peptide désiré à partir de la culture ainsi préparée.

18. Formulation pharmaceutique comprenant un peptide selon l'une des revendications 1 à 3 associé à un support acceptable d'un point de vue physiologique.

19. Formulation selon la revendication 18 adaptée à une administration par injection.

20. Formulation selon la revendication 18 adaptée à une administration topique.

21. Formulation selon la revendication 18 adaptée à une administration par instillation dans l'oeil.

22. Formulation selon la revendication 18 sous la forme d'un implant chirurgical.

23. Formulation selon l'une des revendications 18 à 22 adaptée à une administration sous forme de doses divisées.

24. Formulation selon l'une des revendications 18 à 23 adaptée à l'administration in vivo d'une dose quotidienne comprise entre 1 µg/kg et 1000 µg/ml environ.

## Revendications (Revendications pour l'(les) Etat(s) contractant(s) suivant(s): ES)

1. Procédé pour la production d'un peptide comprenant une lymphotoxine physiologiquement active chez l'homme et qui ne possède pas le 27mère de l'extrémité N de la molécule naturelle;
ledit procédé comprenant les étapes consistant à exprimer une séquence ou un vecteur codant pour le peptide dans un système d'expression approprié permettant l'expression du peptide et
à purifier le peptide exprimé désiré.

2. Procédé selon la revendication 1 dans lequel le peptide préparé possède la séquence suivante: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 dans laquelle
N et C représentent les extrémités amino et carboxy respectivement, et R représente un résidu méthionine ou N-formylméthionine ou un atome d'hydrogène.

3. Procédé selon la revendication 2 dans lequel R dans le peptide préparé représente un atome d'hydrogène.

4. Séquence nucléotidique codant pour un peptide selon l'une des revendications précédentes.

5. Séquence nucléotidique construite en retour à partir du peptide de l'une des revendications 1 à 3 et codant pour ce peptide.

6. Séquence nucléotidique codant pour un peptide défini à la revendication 1 possédant la séquence suivante:

5'          AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG          3'

7. Séquence selon l'une des revendications 4 à 6 dans laquelle la séquence est une séquence d'ADN.

8. Séquence complémentaire d'une séquence nucléotidique selon l'une des revendications 4 à 7.

9. Séquence nucléotidique selon l'une des revendications 4 à 7 associée de manière opérationnelle avec au moins une séquence de régulation.

10. Séquence nucléotidique selon la revendications 9 dans laquelle au moins une séquence de régulation comprend au moins un opérateur, promoteur, signal de terminaison, inducteur ou toute combinaison appropriée de ces séquences.

11. Vecteur comprenant une séquence nucléotidique selon l'une des revendications 4 à 10.

12. Vecteur selon la revendication 11 dans lequel le vecteur parental est pTLym ou un de ses mutants ou variants.

13. Cellule hôte transformée par une séquence nucléotidique ou par un vecteur selon l'une des revendications 4 à 12, la cellule étant compétente pour la réplication de la séquence ou du vecteur.

14. Cellule hôte selon la revendication 13, la cellule étant une cellule eucaryote.

15. Cellule hôte selon la revendication 13, la cellule étant une cellule procaryote.

16. Cellule hôte selon l'une des revendications 13 à 15, la cellule étant E. coli, saccharomyces, streptomyces, B. subtilis, ou une lignée de cellules d'ovaire de hamster chinois.

17. Procédé pour la production d'un peptide possédant l'activité de la lymphotoxine humaine comprenant les étapes consistant à préparer une culture de cellules selon l'une des revendications 13 à 16 et à purifier le peptide désiré à partir de la culture ainsi préparée.

18. Procédé pour la préparation d'une formulation pharmaceutique comprenant l'association d'un peptide selon l'une des revendications 1 à 3 avec un support acceptable d'un point de vue physiologique.

19. Utilisation d'un peptide défini à l'une des revendications 1 à 3 dans la préparation d'un médicament antitumoral.

20. Procédé ou utilisation selon la revendication 19 dans lequel la formulation ou le médicament est adapté à une administration par injection.

21. Procédé ou utilisation selon la revendication 19 dans lequel la formulation ou le médicament est adapté à une administration topique.

22. Procédé ou utilisation selon la revendication 19 dans lequel la formulation ou le médicament est adapté à une administration par instillation dans l'oeil.

23. Procédé ou utilisation selon la revendication 19 dans lequel la formulation ou le médicament est sous la forme d'un implant chirurgical.

24. Procédé ou utilisation selon l'une des revendications 19 à 23 dans lequel la formulation ou le médicament est adapté à une administration sous forme de doses divisées.

25. Procédé ou utilisation selon l'une des revendications 19 à 24 dans lequel la formulation ou le médicament est adapté à une administration quotidienne in vivo d'entre 1 µg/kg et 1000 µg/ml environ.

## Revendications (Revendications pour l'(les) Etat(s) contractant(s) suivant(s): GR)

1. Procédé pour la production d'un peptide comprenant une lymphotoxine physiologiquement active chez l'homme,qui ne possède pas le 27mère de l'extrémité N de la molécule naturelle;
ledit procédé comprenant les étapes consistant à exprimer une séquence ou un vecteur codant pour le peptide dans un système d'expression approprié permettant l'expression du peptide et
à purifier peptide exprimé désiré.

2. Procédé selon la revendication 1 dans lequel la séquence préparée possède la séquence suivante: 
(N)--R-LysProAlaAlaHisLeuIleGlyAspProSerLysGlnAsn
SerLeuLeuTrpArgAlaAsnThrAspArgAlaPheLeuGlnAsp
GlyPheSerLeuSerAsnAsnSerLeuLeuValProThrSerGly
IleTyrPheValTyrSerGlnValValPheSerGlyLysAlaTyr
SerProLysAlaThrSerSerProLeuTyrLeuAlaHisGluVal
GlnLeuPheSerSerGlnTyrProPheHisValProLeuLeuSer
SerGlnLysMetValTyrProGlyLeuGlnGluProTrpLeuHis
SerMetTyrHisGlyAlaAlaPheGlnLeuThrGlnGlyAspGln
LeuSerThrHisThrAspGlyIleProHisLeuValLeuSerPro
SerThrValPhePheGlyAlaPheAlaLeu--(C)
 dans laquelle
N et C représentent les extrémités amino et carboxy respectivement et R représente un résidu méthionine ou N-formylméthionine ou un atome d'hydrogène.

3. Procédé selon la revendication 2 dans lequel R dans le peptide préparé représente un atome d'hydrogène.

4. Procédé selon l'une des revendications précédentes dans lequel la séquence codant pour le nucléotide est construite en retour à partir du peptide selon l'une des revendications précédentes et code pour ce peptide.

5. Procédé selon l'une des revendications précédentes dans lequel la séquence nucléotidique possède la séquence suivante:

5'          AAA CCT GCA GCT CAC CTC ATT GGC
GAT CCG TCT AAG CAG AAT TCA CTA CTG TGG
AGA GCA AAC ACG GAC CGT GCC TTC CTC CAG
GAT GGC TTC TCC TTG TCC AAC AAC TCT TTG
CTG GTA CCG ACC AGT GGC ATC TAC TTC GTG
TAC TCT CAG GTG GTT TTT AGC GGG AAA GCT
TAC AGC CCG AAG GCC ACC TCT TCC CCG CTG
TAT CTG GCG CAT GAG GTC CAA CTG TTC TCG
AGC CAA TAT CCG TTC CAC GTG CCA CTC CTG
AGC TCC CAG AAG ATG GTT TAC CCC GGG CTT
CAA GAA CCG TGG CTG CAC AGC ATG TAC CAT
GGG GCG GCG TTT CAG TTA ACC CAA GGT GAC
CAA CTA TCT ACT CAC ACA GAC GGG ATC CCG
CAC CTA GTC CTC AGC CCG AGT ACT GTC TTC
TTT GGA GCC TTC GCT CTG          3'

6. Procédé selon la revendication 4 ou 5 dans lequel la séquence est une séquence d'ADN.

7. Procédé selon l'une des revendications 1 à 6 dans lequel la séquence nucléotidique codante est associée de manière opérationnelle avec au moins une séquence de régulation.

8. Procédé selon la revendication 7 dans laquelle au moins une séquence de régulation comprend au moins un opérateur, promoteur, signal de terminaison, inducteur ou toute combinaison appropriée de ces séquences.

9. Procédé selon l'une des revendications précédentes dans lequel les cellules sont transformées par un vecteur comprenant une séquence nucléotidique selon l'une des revendications 4 à 8.

10. Procédé selon la revendication 9 dans lequel le vecteur parental est pTLym ou un de ses mutants ou variants.

11. Cellule hôte transformée avec une séquence nucléotidique ou un vecteur selon l'une des revendications 4 à 10, la cellule étant compétente pour la réplication de la séquence ou vecteur.

12. Cellule hôte selon la revendication 11, la cellule étant une cellule eucaryote.

13. Cellule hôte selon la revendication 11, la cellule étant une cellule procaryote.

14. Cellule hôte selon l'une des revendications 11 à 13, la cellule étant E. coli, saccharomyces, streptomyces, B. subtilis, ou une lignée de cellules d'ovaire de hamster chinois.

15. Procédé pour la production d'un peptide possédant l'activité de la lymphotoxine humaine comprenant les étapes consistant à préparer une culture de cellules selon l'une des revendications 11 à 14 et à purifier le peptide désiré à partir de la culture ainsi préparée.

16. Procédé pour la préparation d'une formulation pharmaceutique comprenant l'association d'un peptide selon l'une des revendications 1 à 3 avec à un support acceptable d'un point de vue physiologique.

17. Utilisation d'un peptide défini à l'une des revendications 1 à 3 dans la préparation d'un médicament antitumoral.

18. Procédé ou utilisation selon la revendication 17 dans lequel la formulation ou le médicament est adapté à une administration par injection.

19. Procédé ou utilisation selon la revendication 17 dans lequel la formulation ou le médicament est adapté à une administration topique.

20. Procédé ou utilisation selon la revendication 17 dans lequel la formulation ou le médicament est adapté à une administration par instillation dans l'oeil.

21. Procédé ou utilisation selon la revendication 17 dans lequel la formulation ou le médicament est sous la forme d'un implant chirurgical.

22. Procédé ou utilisation selon l'une des revendications 17 à 21 dans lequel la formulation ou le médicament est adapté à une administration sous forme de doses divisées.

23. Procédé ou utilisation selon l'une des revendications 17 à 22 dans lequel la formulation ou le médicament est adapté à une administration quotidienne in vivo comprise entre 1 µg/kg et 1000 µg/ml environ.
